# EUROPEAN PATENT APPLICATION

(11) **EP 2 786 739 A1**
(43) Date of publication of application: **08.10.2014**
(21) Application number: 12853918.6
(22) Date of filing: 28.11.2012
(51) Int. Cl.: A61K 8/34, A61K 8/46, A61Q 5/02, C11D 1/02, C11D 3/20, C11D 3/37

(54) **HAIR CLEANING AGENT COMPOSITION**

(30) Priority: 28.11.2011 JP 2011259447
(71) Applicant: Kao Corporation, Chuo-Ku Tokyo 103-8210 (JP)
(72) Inventor: TAKIGUCHI, Osamu, Tokyo 131-8501 (JP); HIRANO, Yuji, Tokyo 131-8501 (JP)
(74) Representative: HOFFMANN EITLE
(86) International application number: PCT/JP2012/080772
(87) International publication number: WO 2013/081017

(57) **Abstract**

A hair cleaning composition comprising components (A), (B), and (D), wherein the content of a component (C) is suppressed to 0% to 0.05% by mass, and the pH value of the hair cleaning composition is from 2.5 to 5.0;
(A): polyglycerin, in which with regard to the ratio among a total mass (p) of triglycerin and tetraglycerin, a mass (q) of diglycerin, and a total mass (r) of pentaglycerin and higher order oligomers, q/p is less than 0.5 and r/p is less than 0.5;
(B): aromatic alcohol;
(C): cationic surfactant; and
(D): anionic surfactant.

## Description

### [Field of the Invention]

The present invention relates to a hair cleaning composition, which reforms the inside of hair and improves hair styling properties.

### [Background of the Invention]

Methods for imparting a desired style to hair and retaining it can be broadly classified into: a surface reformation method comprising allowing a hair styling component to remain on the surface of hair; and an internal reformation method comprising penetrating a hair styling component into the inside of hair. The former method is characterized in that it easily provides high hair styling properties, but it tends to provide unnatural hand feeling. On the other hand, the latter method is characterized in that it hardly provides sufficient hair styling properties, but it tends to provide natural hand feeling.

As a result of the recent trend in hairstyle, preference for natural hairstyle that is not too elaborated and good hand feeling has increased. In such background, it has been desired to develop a technique of reforming the inside of hair that provides higher hair styling effects, while keeping natural hand feeling and/or finishing.

As a technique of improving hair styling properties by reforming the inside of hair, a technique of reforming the inside of hair by allowing sugar, amino acid, enzyme or the like to penetrate into hair has been known (Patent Literatures 1 and 2). Also, it has been reported that polyglycerin having a specific molecular weight or a specific polymerization degree has excellent hair styling properties, although it has not been suggested that such polyglycerin has an internal reforming effect (Patent Literatures 3 and 4).

Moreover, in recent years, there has been a trend in which hair color is changed by a color-treatment for fashion, or hair style is changed by an entire or partial perm treatment. As a result, hair is largely damaged by such chemical treatments or daily hair washing. Under such circumstances, a hair styling agent, in which a conditioning component is combined with a glycerin derivative, has been proposed (Patent Literature 5).

### [Citation List]

### [Patent Literature]

[Patent Literature 1] JP-A-2007-131581
[Patent Literature 2] JP-A-2009-108085
[Patent Literature 3] JP-A-2001-97825
[Patent Literature 4] JP-A-2003-286139
[Patent Literature 5] JP-A-2005-2038

### [Summary of the Invention]

The present invention provides a hair cleaning composition comprising components (A) and (B), wherein the content of a component (C) is suppressed to 0% to 0.05% by mass, and the pH value of hair cleaning composition is from 2.5 to 5.0;
(A): polyglycerin, in which with regard to the ratio among a total content (p) of triglycerin and tetraglycerin, a content (q) of diglycerin, and a total content (r) of pentaglycerin and higher order oligomers in the entire polyglycerin, q/p is less than 0.5 and r/p is less than 0.5;
(B): aromatic alcohol;
(C): cationic surfactant; and
(D): anionic surfactant.

The present invention also provides a hair styling method, which comprises applying the aforementioned hair cleaning composition to hair, rinsing it from the hair, and then styling the hair, while blow-drying it with hot air from a dryer.

### [Embodiments for carrying out the Invention]

At the conventional technical level, a technique of reforming the inside of hair, which is capable of improving hair styling properties to the adequate level while keeping natural hand feeling, has not yet been discovered. The present inventors have found that, when polyglycerin known to have an excellent hair styling properties is used in combination with a cationic surfactant used as a conditioning component, its hair styling properties is impaired, and when such polyglycerin is used in a large amount in order to retain such hair styling properties, stickiness is generated. Thus, polyglycerin has a trade-off problem. Moreover, the present inventors have also found that both hair styling properties and good feeling can be achieved while suppressing the amount of polyglycerin mixed, when polyglycerin, the polymerization degree distribution of which is regulated in a certain range, is incorporated into a hair cleaning agent comprising aromatic alcohol, having a pH value which is adjusted to be low, and also comprising substantially no cationic surfactant.

### [(A): Polyglycerin]

Polyglycerin is generally produced and sold in the form of a mixture of polymers ranging from dimers to nonamers. The present inventors have found that while trimers (triglycerin) and tetramers (tetraglycerin) contained in polyglycerin have an excellent internal reforming effect, polymers such as dimers (diglycerin), pentamers, and higher order oligomers (pentaglycerin, heptaglycerin, etc) do not have such an internal reforming effect. Moreover, the inventors have found that diglycerin with a low molecular weight easily penetrates into hair to occupies a site in which triglycerin or tetraglycerin could exhibit effects, and that diglycerin thus becomes an inhibitory factor which inhibits the achievement of an internal reforming effect. The inventors have also found that polymers with a high molecular weight, such as pentaglycerin or heptaglycerin, hardly penetrate into hair, and that thus it remains on the surface and causes a reduction in good feeling. Hence, the conventionally used polyglycerin comprises large quantities of dimers, pentamers and higher order oligomers, and as a result, sufficient hair styling effects cannot be obtained. For example, only the optimization of the mixing ratio of a cationic surfactant and the like and selection of the type of such a surfactant, as described later, are insufficient for obtaining sufficient hair styling effects.

Herein, the term "diglycerin" is used to mean a dimer in which two molecules of glycerins bind to each other in a linear or cyclic form as a result of dehydration synthesis. The diglycerin has, for example, the form of the following compounds (2-1) or (2-2):

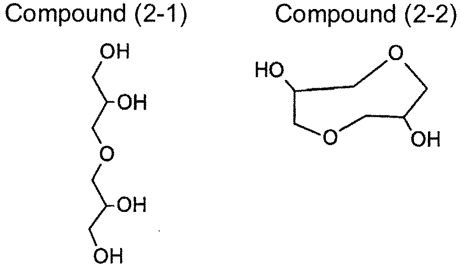

The term "triglycerin" is used herein to mean a trimer in which three molecules of glycerins bind to one another in a linear, cyclic or branched form as a result of dehydration synthesis. The triglycerin has, for example, the form of the following compounds (3-1) to (3-5) :

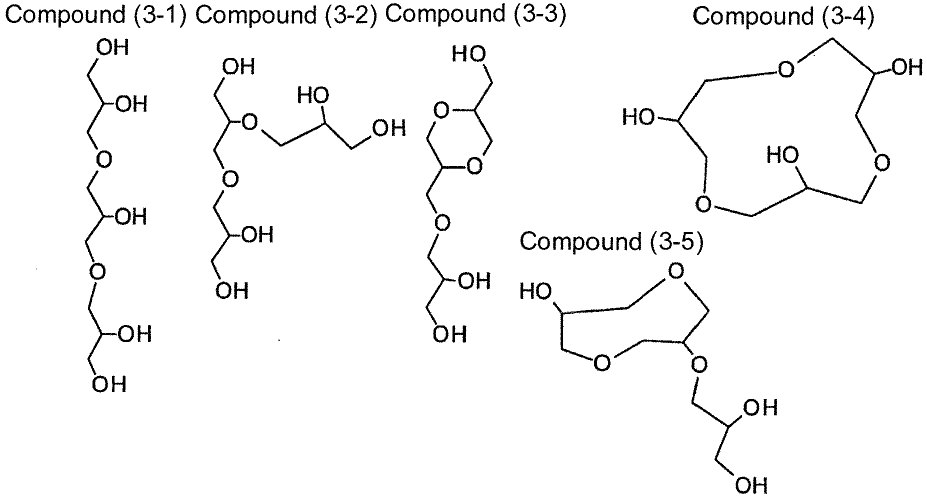

The term "tetraglycerin" is used herein to mean a tetramer in which four molecules of glycerins bind to one another in a linear, cyclic or branched form as a result of dehydration synthesis. The tetraglycerin has, for example, the form of the following compounds (4-1) to (4-9) :

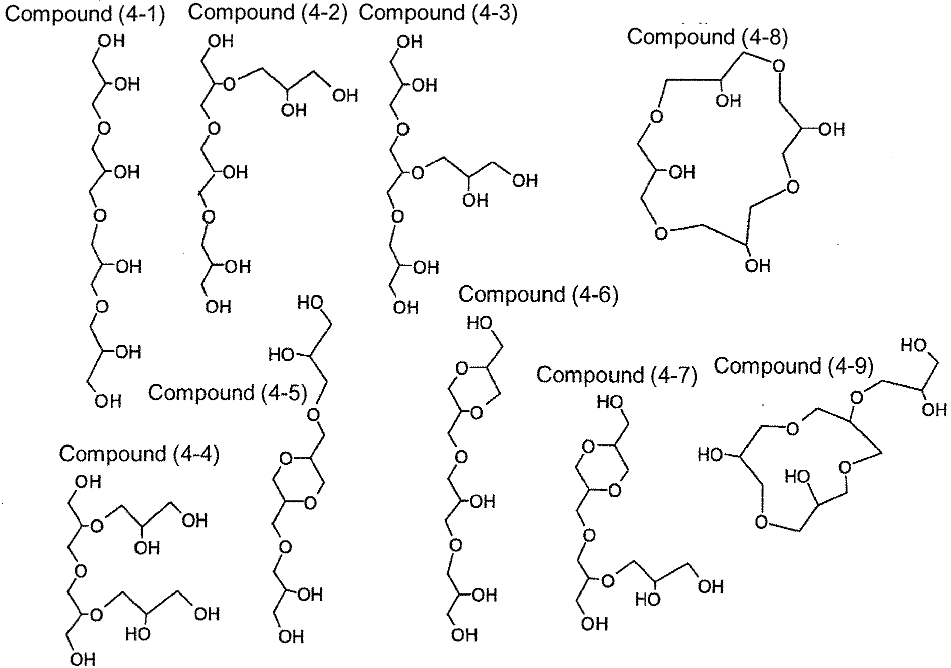

From the viewpoint of the achievement of excellent hair styling properties, with regard to polyglycerin as component (A), the ratio of the content (q) of dimers to the total content (p) of trimers and tetramers in the entire polyglycerin, (q)/(p), is set at less than 0.5, preferably less than 0.4, and more preferably less than 0.3. On the other hand, from the viewpoint of feeling obtained after polyglycerin has been applied to hair, the ratio of the total content (r) of pentamer and higher order oligomers to the total content (p) of trimers and tetramers in the entire polyglycerin, (r)/(p), is set at less than 0.5, preferably less than 0.4, and more preferably less than 0.2. Polyglycerin having such polymerization degree distribution has a hydroxyl value of approximately 970 to 1242 (mgKOH/g), and preferably 1000 to 1200 (mgKOH/g). Furthermore, the above described polyglycerin has a specific gravity at 30°C of 1.271 to 1.350, and preferably 1.275 to 1.320. These physical values may be used as subsidiary grounds for selecting component (A).

As such polyglycerin, two or more polyglycerins may be used in combination. In such a case, the entire polyglycerin as component (A) is required to fulfill the above described conditions. Examples of a commercially available polyglycerin that fulfills the above described conditions include polyglylcerol-3 (q/p = 0.44, r/p = 0.13, hydroxyl value: 1140, specific gravity: 1.285) and polyglylcerol-4 (q/p = 0.03, r/p = 0.37, hydroxyl value: 1053, specific gravity: 1.296), both of which are manufactured by Solvay.

The mass ratio among individual components in polyglycerin can be obtained by the following method. Specifically, trimethylsilylation is performed on a polyglycerin mixture as a measurement target so as to convert it to a polyglycerin derivative, and thereafter, the polyglycerin derivative is subjected to separation and quantification according to a GC method (gas chromatography), and the mass ratio can be then determined according to an area method. The analysis according to the GC method can be easily carried out by analyzing temperature rising from 100°C to 320°C at a rate of 10°C/min, using a fused silica capillary tube, to which a low-polarity liquid phase such as methyl silicone has been allowed to chemically bind. In addition, the hydroxyl value can be determined, for example, according to the method described in JIS K 1557-1. Specific gravity can be determined according to First Method A described in Japanese Standards of Quasi-drug Ingredients 2006.

The value (p) in polyglycerin as component (A) in the composition of the present invention is preferably from 52% to 100% by mass, and more preferably from 55% to 100% by mass, from the viewpoint of suppressing stickiness, while keeping excellent hair styling effects caused by hair internal reforming.

The content of polyglycerin as component (A) in the composition of the present invention is preferably from 0.1% to 10% by mass, more preferably from 0.3% to 5% by mass, and even more preferably from 0.5% to 2.5% by mass, from the viewpoint of suppressing stickiness, while keeping excellent hair styling effects caused by hair internal reforming.

### [(B): Aromatic alcohol]

Aromatic alcohol as component (B) is used from the viewpoint of promoting permeation of the polyglycerin used as a component (A) that is an internal reforming component into hair. Examples of such aromatic alcohol include benzyl alcohol, phenoxyethanol, and 2-benzyloxyethanol. These substances can be used singly or in combination of two or more substances.

The content of the aromatic alcohol used as a component (B) in the composition of the present invention is preferably from 0.05% to 7.0% by mass, more preferably from 0.1% to 5.0% by mass, and even more preferably from 0.2% to 3.0% by mass.

### [(C): Cationic surfactant]

It is necessary for the hair cleaning composition of the present invention to comprise substantially no cationic surfactant which is an inhibitory factor to hair styling properties provided by polyglycerin, namely, that the content of the cationic surfactant is suppressed to 0% to 0.05% by mass. In order for the hair cleaning composition of the present invention to have excellent hair styling properties, it is preferable that the present hair cleaning composition comprise completely no cationic surfactant (content: 0% by mass). However, if the present composition comprises a trace amount of cationic surfactant, a reduction in hair styling properties caused thereby is ignorable. Thus, the permissible limit is set at 0.05% by mass. A more preferred permissible limit of the cationic surfactant is less than 0.03% by mass, and more preferably less than 0.01% by mass.

Examples of the cationic surfactant whose content is limited in the present invention include tertiary amines such as alkyl dimethyl amine, etheramine and amideamine, which are used via formation of acid-added salts, as well as quaternary ammonium salts.

### [(D): Anionic surfactant]

The hair cleaning composition of the present invention comprises an anionic surfactant.

As such anionic surfactants, sulfuric acid-based surfactants, sulfonic acid-based surfactants, and carboxylic acid-based surfactants can be used. Examples of such a sulfuric acid-based surfactant include, polyoxyalkylene alkenyl ether sulfate, and sulfosuccinic acid alkylene alkylphenyl ether sulfate. Moreover, sulfosuccinic acid alkyl ester salts, polyoxyalkylenesulfosuccinic acid alkyl ester salts, higher fatty acid salts, alkanesulfonic acid salts and the like can be used. Among them, polyoxyalkylene alkyl ether sulfate and alkyl sulfate are preferable, and those represented by the following general formula (1) or (2) are preferable.

R¹O-(CH₂CH₂O)ₐSO₃M (1)

R²O-SO₃M (2)

R¹ represents an alkyl group or alkenyl group containing 10 to 18 carbon atoms; R² represents an alkyl group containing 10 to 18 carbon atoms; M represents alkaline metal, alkaline-earth metal, ammonium, alkanolamine or basic amino acid; and a represents a mass average which is a number of 1 to 5.

In terms of foaming properties, and liquid properties and cleaning properties upon application, the content of such an anionic surfactant is preferably 60% by mass or less, more preferably 30% by mass or less, and even more preferably 20% by mass or less. Also, it is preferably 0.1% by mass or more, more preferably 1% by mass or more, and even more preferably 5% by mass or more. That is, it is preferably from 0.1% to 30% by mass, more preferably from 0.5% to 25% by mass, even more preferably from 1% to 20% by mass, and further preferably 5% to 20% by mass.

### [Other surfactants]

The hair cleaning composition of the present invention may comprise any one or more of surfactants other than (C) the cationic surfactant and (D) the anionic surfactant, namely, any one or more of either amphiphilic surfactants or nonionic surfactants.

Examples of the amphiphilic surfactant include betaine-based surfactants. Among such betaine-based surfactants, betaine-based surfactants such as alkyldimethylaminoacetic acid betaines and fatty acid amide propyl betaines are preferable. Of these, fatty acid amide propyl betaines are preferable. As such fatty acid amide propyl betaines, fatty acid amide propyl betaines having an acyl group containing 8 to 18, or preferably 10 to 16 carbon atoms, are preferable. Among them, lauric acid amide propyl betaine, palm kernel oil fatty acid amide propyl betaine, and coconut oil fatty acid amide propyl betaine and the like are preferable.

Examples of the nonionic surfactant include polyoxyalkylene alkyl ether, polyoxyalkylene alkenyl ether, higher fatty acid sucrose ester, polyglycerin fatty acid ester, higher fatty acid mono- or di-ethanolamide, polyoxyethylene hydrogenated castor oil, polyoxyethylene sorbitan fatty acid ester, polyoxyethylene sorbit fatty acid ester, alkyl saccharide, alkyl amine oxide, alkyl amide amine oxide, alkyl glyceryl ether, and alkenyl glyceryl ether. Among these, polyoxyalkylene alkyl ether, higher fatty acid mono- or di-ethanolamide, polyoxyethylene hydrogenated castor oil, and alkyl glyceryl ether are preferable. Polyoxyethylene alkyl (average number of carbon atoms: 12 to 14) ether, 2-ethylhexyl glyceryl ether, and isodecyl glyceryl ether are preferable.

In terms of foaming properties, and liquid properties and cleaning properties upon application, the content of surfactants other than the cationic surfactant and the anionic surfactant in the hair cleaning composition of the present invention is preferably from 0% to 40% by mass, more preferably from 0.5% to 30% by mass, and even more preferably from 1% to 25% by mass.

### [Cationic polymer]

Moreover, from the viewpoint of the improvement of smoothness upon application of the composition to hair and upon drying of the hair, the hair cleaning composition of the present invention may further comprise a cationic polymer. Examples of such a cationic polymer include a cationized cellulose derivative, cationic starch, a cationized guar gum derivative, a homopolymer of diallyl quaternary ammonium salts, a diallyl quaternary ammonium salt/acylamide copolymer, a quaternized polyvinylpyrrolidone derivative, a polyglycol polyamine condensate, a vinyl imidazolium trichloride/vinylpyrrolidone copolymer, a hydroxyethylcellulose/dimethyl diallyl ammonium chloride copolymer, a vinylpyrrolidone/quaternized dimethylaminoethyl methacrylate copolymer, a polyvinylpyrrolidone/alkylamino acrylate copolymer, a polyvinylpyrrolidone/alkylamino acrylate/vinyl caprolactam copolymer, a vinylpyrrolidone/methacrylamide propyl trimethylammonium chloride copolymer, an alkyl acrylamide/acrylate/alkylaminoalkyl acrylamide/polyethylene glycol methacrylate copolymer, an adipic acid/dimethylaminohydroxypropyl ethylene triamine copolymer (Cartaretin manufactured by Sandoz Ltd., U. S. A.), and cationic polymers described in JP-A-53-139734 and JP-A-60-36407. Among them, a cationized cellulose derivative, a cationized guar gum derivative, and a diallyl quaternary ammonium salt/acrylamide copolymer are preferable. These substances can be used singly or in combination of two or more substances.

The content of the cationic polymer in the composition of the present invention is preferably from 0.02% to 5% by mass, more preferably from 0.05% to 1% by mass, and even more preferably from 0.1% to 0.7% by mass, in terms of the improvement of the manageability of hair after drying and feeling after drying.

### [Silicone]

Furthermore, in terms of smoothness upon application to hair and upon drying of the hair, the hair cleaning composition of the present invention may further comprise silicone. Examples of such silicone are as follows.

### (1) Dimethyl polysiloxane

Dimethyl polysiloxane represented by the following general formula may be used, for example:

(CH₃)₃SiO-[(CH₃)₂SiO]_{d}-Si(CH₃)₃

wherein d represents a number of 3 to 20000.

### (2) Amino-modified silicone

Various types of amino-modified silicones can be used. Among them, amino-modified silicone having a mean molecular weight of approximately 3,000 to 100,000, which is described with the name "Amodimethicone" in the 3^{rd} edition of CTFA Dictionary (Cosmetic Ingredient Dictionary, U. S. A.), is preferable. This amino-modified silicone is preferably used in the form of an aqueous emulsion, and commercially available products of this amino-modified silicone include SM 8704C (Dow Corning Toray Co., Ltd.), DC 929 (Dow Corning), and KT 1989 (Momentive Performance Materials, Inc.).

### (3) Other silicones

In addition to the above-mentioned silicons, examples of other silicones include polyether-modified silicone, methylphenyl polysiloxane, fatty acid-modified silicone, alcohol-modified silicone, alkoxy-modified silicone, epoxy-modified silicone, fluorine-modified silicone, cyclic silicone, and alkyl-modified silicone. These substances can be used singly or in combination of two or more substances.

The content of the silicone in the composition of the present invention is preferably from 0.01% to 10% by mass, more preferably from 0.05% to 6% by mass, and even more preferably from 0.3% to 3% by mass, in terms of smoothness from application to hair until rinsing the hair.

### [Oil agent]

Further, the hair cleaning composition of the present invention may also comprise an oil agent as another conditioning agent. Examples of such an oil agent include: hydrocarbons such as squalene, squalane, liquid paraffin, liquid isoparaffin and cycloparaffin; glycerides such as castor oil, cacao oil, mink oil, avocado oil and olive oil; waxes such as beeswax, spermaceti, lanolin and carnauba wax; polyhydric alcohols such as glycerin; esters such as isopropyl palmitate, isopropyl myristate, octyl dodecyl myristate, hexyl laurate, cetyl lactate, propylene glycol monostearate, oleyl oleate, hexadecyl 2-ethylhexanoate, isononyl isononanoate, and tridecyl isononanoate; higher fatty acids such as capric acid, lauric acid, myristic acid, palmitic acid, stearic acid, behenic acid, oleic acid, coconut oil fatty acid, isostearyl acid, and isopalmitic acid; higher alcohols such as myristyl alcohol, cetyl alcohol, stearyl alcohol, behenyl alcohol, 2-octyldodecanol, and cetostearyl alcohol; other oil agents such as isotearyl glyceryl ether; and polyoxy propylene butyl ether. Among these, higher alcohols are preferable, and in particular, myristyl alcohol, cetyl alcohol, and stearyl alcohol are preferable. These oil agents may also be used in combination of two or more oil agents. The content of the oil agent in the hair cleaning composition of the present invention is preferably from 0.2% to 2% by mass, more preferably from 0.3% to 1.8% by mass, and even more preferably from 0.5% to 1.5% by mass.

### [Medium]

In the hair cleaning composition of the present invention, water and, as necessary, an organic solvent other than the component (B) are used as media. Examples of such an organic solvent include: lower alkanols such as ethanol and 2-propanol; polyols such as propylene glycol, 1,3-butanediol, diethylene glycol and glycerin; cellosolves such as ethyl cellosolve and butyl cellosolve; and carbitols such as ethyl carbitol and butyl carbitol.

The content of water in the hair cleaning composition of the present invention is preferably 50% by mass or more, more preferably from 60% to 99% by mass, and even more preferably from 70% to 97% by mass.

### [Other components]

In addition to the above-mentioned components, the hair cleaning composition of the present invention may also comprise other components generally used as raw materials for cosmetics. Examples of such components include antiseptics, chelating agents, stabilizers, antioxidants, plant extracts, herbal extracts, protein hydrolysates, vitamins, coloring agents such as dyes, perfumes, ultraviolet absorbers, pearling agents such as ethylene glycol difatty acid ester, polymers for hair setting, and amphiphilic amide lipids.

### [pH]

From the viewpoint of promoting permeation of polyglycerin as component (A) that is an internal reforming component into the inside of hair, the pH value of the hair cleaning composition of the present invention at 25°C is preferably 2.5 to 5.0, and more preferably 3.0 to 4.5.

In order to adjust the pH value of the hair cleaning composition of the present invention within the aforementioned range, it is preferable to use organic acid, and it is more preferable to use hydroxycarboxylic acid. Specific examples of such hydroxycarboxylic acid include glycolic acid, lactic acid, malic acid, tartaric acid, citric acid, gluconic acid, and pantothenic acid. The amount of the organic acid used is arbitrarily determined, such that the pH value of the hair cleaning composition of the present invention can be set within the aforementioned range.

### [Method for treating hair]

The hair cleaning composition of the present invention can be used for treating the hair by applying the hair cleaning composition to hair, washing the hair, rinsing it from the hair, and then drying the hair. In addition, this drying may be natural drying, but in order to impart sufficient hair styling properties to hair, it is preferable to style hair while drying the hair with hot air from a dryer. By this treatment, hair can be preferably styled.

### [Method for re-styling hair]

The hair cleaning composition of the present invention can be used for re-styling, after completion of the above described hair treatment, which comprises applying the hair cleaning composition to hair, washing the hair, rinsing it from the hair, and then drying the hair. That is to say, after the hair has been dried by natural drying or with hot hair from a dryer, the hair can be styled, immediately or after a certain time period, such as after leaving overnight. When hair is re-styled, water, a solvent or the like may be first put on the hair to get it wet, and the hair may be then re-styled. In the present invention, however, it is not always necessary to get hair wet. Then, hair can be styled by heating the hair using a heat generation type hair styling device such as a dryer, a hot curler, electric hair tongs or an iron.

With regard to the above-mentioned embodiments, preferred aspects of the present invention will be further disclosed below.

### < 1 >

A hair cleaning composition comprising components (A) and (B), wherein the content of a component (C) is suppressed to 0% to 0.05% by mass, and the pH value of the hair cleaning composition is from 2.5 to 5.0;
(A): polyglycerin, in which with regard to the ratio among a total content (p) of triglycerin and tetraglycerin, a content (q) of diglycerin, and a total content (r) of pentaglycerin and higher order oligomers in the entire polyglycerin, q/p is less than 0.5 and r/p is less than 0.5;
(B): aromatic alcohol;
(C): cationic surfactant; and
(D): anionic surfactant.

### < 2 >

The hair cleaning composition according to < 1 > above, wherein the content of the component (A) is preferably from 0.1% to 10% by mass, more preferably from 0.3% to 5% by mass, and even more preferably from 0.5% to 2.5% by mass.

### < 3 >

The hair cleaning composition according to < 1 > or < 2 > above, wherein q/p in the component (A) is preferably less than 0.4, and more preferably less than 0.3.

### < 4 >

The hair cleaning composition according to any one of < 1 > to < 3 > above, wherein r/p in the component (A) is preferably less than 0.4, and more preferably less than 0.2.

### < 5 >

The hair cleaning composition according to any one of < 1 > to < 4 > above, wherein the content of the component (B) is preferably from 0.05% to 7.0% by mass, more preferably from 0.1% to 5.0% by mass, and even more preferably from 0.2% to 3.0% by mass.

### < 6 >

The hair cleaning composition according to any one of < 1 > to < 5 > above, wherein the content of the component (C) is preferably less than 0.03% by mass, more preferably less than 0.01% by mass.

### < 7 >

The hair cleaning composition according to any one of < 1 > to < 6 > above, wherein the content of the component (D) is preferably from 0.1% to 30% by mass, more preferably from 0.5% to 25% by mass, even more preferably from 1% to 20% by mass, even more preferably from 5% to 20% by mass.

### < 8 >

The hair cleaning composition according to any one of < 1 > to < 7 > above, wherein the anionic surfactant is at least one selected from the group consisting of polyoxyalkylene alkyl ether sulfate and alkyl sulfate.

### < 9 >

A method for treating hair, which comprises applying the hair cleaning composition according to any one of < 1 > to < 8 > above to hair, washing the hair, rinsing it from the hair, and then drying the hair by natural drying or by blow-drying with hot air from a dryer.

### < 10 >

A method for treating hair, which comprises applying the hair cleaning composition according to any one of < 1 > to < 8 > above to hair, washing the hair, rinsing it from the hair, then drying the hair by natural drying or by blow-drying with hot air from a dryer, and then styling the hair by heating it using a heat generation type hair styling device such as a dryer, a curler, electric hair tongs or an iron.

### < 11 >

Use of the hair cleaning composition according to any one of < 1 > to < 8 > above for treating hair, which comprises applying the hair cleaning composition according to any one of < 1 > to < 8 > above to hair, washing the hair, rinsing it from the hair, and then drying the hair by natural drying or by blow-drying with hot air from a dryer.

### < 12 >

Use of the hair cleaning composition according to any one of < 1 > to < 8 > above for re-styling hair, which comprises applying the hair cleaning composition according to any one of < 1 > to < 8 > above to hair, washing the hair, rinsing it from the hair, then drying the hair by natural drying or by blow-drying with hot air from a dryer, and then styling the hair by heating it using a heat generation type hair styling device such as a dryer, a curler, electric hair tongs or an iron.

### [Examples]

### Examples 1 to 3 and Comparative Examples 1 to 5

Hair shampoos shown in Table 3 were prepared, and the hair styling properties of each hair shampoo to damaged hair was then evaluated in accordance with the following methods and standards.

### < Preparation of damaged hair >

5 g of hair tress was prepared from chemically untreated, Japanese unwanted fuzzy hair, and the following permanent treatment A was performed on the hair tress once. Thereafter, a series of bleaching operations B, as described below, were performed thereon five times, so as to prepare damaged hair.

- Permanent treatment A: Pre-Mina Wave Lotion (hard type) manufactured by Kao Professional Services Co., Ltd. was used. First, a first part was applied to the hair tress at a bath ratio of 1 : 1, and it was then left at a room temperature for 30 minutes, followed by rinsing it from the hair tress with running water. Subsequently, a second part was applied to the hair tress at a bath ratio of 1 : 1, and it was then left at a room temperature for 30 minutes, followed by rinsing it from the hair tress with running water. Upon performing these operations, in order to keep the original unruly shape, the operations were carried out such that a load was not applied on the hair tress. As a finishing operation, the hair tress was washed with a shampoo for evaluation shown in Table 1. Thereafter, it was treated with a conditioner for evaluation shown in Table 2, and the conditioner was then rinsed from the hair tress, followed by blow-drying.

Thereafter, a series of operations such as washing with the shampoo, treatment with the conditioner, and blow-drying were carried out repeatedly 15 times.

- Bleaching treatment B: a first part and a second part of Puritia Funwari Awa Bleach (high bleach) manufactured by Kao Corporation were mixed with each other, and the obtained mixture was applied to the hair tress at a bath ratio of 1 : 1. Thereafter, it was left at a room temperature for 30 minutes. After the agent was fully rinsed with running water, the hair was washed with the shampoo for evaluation shown in Table 1, and it was treated with the conditioner for evaluation shown in Table 2. Then, the conditioner was rinsed from the hair, and the resulting hair was blow-dried.

Thereafter, a series of operations such as washing with the shampoo, treatment with the conditioner, and blow-drying were carried out repeatedly 15 times.

**[Table 1]**

| Shampoo for evaluation | Mixed amount (% by mass) |
|---|---|
| Ion exchange water | 82.40 |
| EDTA-2Na (*1) | 0.30 |
| Na benzoate aqueous solution (35% by mass) | 1.43 |
| Lauramide DEA (*2) | 1.50 |
| Na laureth sulfate (*3) | 14.35 |
| Phosphoric acid | 0.02 |
| Total | 100.00 |

| | |
|---|---|
| *1:Clewat N, manufactured by Nagase ChemteX Corporation *2:AMINON L-02, manufactured by Kao Corporation *3:EMAL 227 (25% by mass aqueous solution), manufactured by Kao Corporation | |

**[Table 2]**

| Conditioner for evaluation | Mixed amount (% by mass) |
|---|---|
| Ion exchange water | 88.83 |
| Methyl p-hydroxybenzoate | 0.1 |
| Cetearyl alcohol (*4) | 2.0 |
| Propylene glycol | 5.0 |
| Distearyldimonium chloride (*5) | 2.7 |
| Isopropyl alcohol | 0.61 |
| Steartrimonium chloride (*6) | 0.76 |
| Total | 100.0 |

| | |
|---|---|
| *4:KALCOL 6870, manufactured by Kao Corporation *5:QUARTAMIN D86P (75% by mass aqueous solution), manufactured by Kao Corporation *6:QUARTAMIN 86W (28% by mass aqueous solution), manufactured by Kao Corporation | |

### < Measurement of hair styling properties >

A portion with a length of 20 mm was cut from a portion with a curl radius of 10 mm in the damaged hair, and eight samples were prepared. Thereafter, 1.0 g of a weight was attached to a tip of each sample, and it was then immersed in ion exchange water for 30 minutes. Subsequently, a tip on a side opposite to the weight was held and drawn up, and the sample was then dried at 75°C for 10 seconds with the weight which was still attached. The sample was left as was at 20°C for 5 minutes. Thereafter, the weight was removed from the sample, and the curl radius r was then measured.

Subsequently, the same sample as used above was washed with the shampoo for evaluation of Table 1, and each shampoo shown in Table 3 was then applied to the sample at a bath ratio of 1 : 1. Then, the sample was retained at 38°C for 30 minutes. Thereafter, the sample was rinsed with running water, and 1.0 g of a weight was linearly set in the same manner as described above. Then, the curl radius r' was measured.

Hair styling properties were defined as [(r'/r) - 1] x 100, and the hair styling properties was then evaluated using a mean value of the eight samples and standard deviation. The results are shown in Table 3.

The higher the hair styling properties, the more preferable it is. The smaller the standard deviation, the higher the homogeneity that can be favorably obtained.

It is to be noted that, in Comparative Example 5, the evaluation results are given, not as an average of the eight samples, but as those obtained from only a single sample.

**[Table 3]**

| Raw material (% by mass) | Example | | | Comparative Example | | | | |
|---|---|---|---|---|---|---|---|---|
| | 1 | 2 | 3 | 1 | 2 | 3 | 4 | 5 |
| Polyglycerin (1) (*7) | 1.0 | 0.5 | | | | | | 1.0 |
| Polyglycerin (2) (*8) | | 0.5 | 1.0 | | | | | |
| Diglycerin (*9) | | | | 1.0 | | | | |
| Polyglycerin (3) (*10) | | | | | 1.0 | | | |
| Polyglycerin (4) (*11) | | | | | | 1.0 | | |
| Benzyl alcohol | 0.3 | 0.3 | 0.3 | 0.3 | 0.3 | 0.3 | | 0.3 |
| Ammonium laureth sulfate solution (*12) | 5.2 | 5.2 | 5.2 | 5.2 | 5.2 | 5.2 | 5.2 | 5.2 |
| Lauramide propyl betaine solution (*13) | 3.33 | 3.33 | 3.33 | 3.33 | 3.33 | 3.33 | 3.33 | 3.33 |
| Isostearyl glyceryl ether solution (*14) | 0.67 | 0.67 | 0.67 | 0.67 | 0.67 | 0.67 | 0.67 | 0.67 |
| Cocamide MEA (*15) | 0.5 | 0.5 | 0.5 | 0.5 | 0.5 | 0.5 | 0.5 | 0.5 |
| Laureth-16 (*16) | 0.7 | 0.7 | 0.7 | 0.7 | 0.7 | 0.7 | 0.7 | 0.7 |
| Liquid malic acid (50% by mass) | 1.1 | 1.1 | 1.1 | 1.1 | 1.1 | 1.1 | 1.1 | 1.1 |
| Potassium hydroxide (48% by mass) | 0.23 | q. s. | 0.23 | 0.23 | 0.23 | 0.23 | q. s. | q. s. |
| Ion exchange water | Balance | Balance | Balance | Balance | Balance | Balance | Balance | Balance |
| Total | 100 | 100 | 100 | 100 | 100 | 100 | 100 | 100 |
| pH(25°C) | 3.9 | 3.3 | 3.9 | 3.9 | 3.9 | 3.9 | 6.8 | 6.8 |
| Hair styling properties (mean value) | 23.0 | 25.6 | 28.2 | 0.0 | 2.6 | 5.1 | 0.0 | 2 |
| Standard deviation of hair styling properties (n = 8) | 6.3 | 9.6 | 11.5 | 12.6 | 8.1 | 9.6 | 16.3 | - |

| | | | | | | | | |
|---|---|---|---|---|---|---|---|---|
| *7 Polyglycerol-3, manufactured by Solvay (q/p = 0.44, r/p = 0.13, hydroxyl value: 1140, specific gravity: 1.285) *8: Polyglycerol-4, manufactured by Solvay (q/p = 0.03, r/p = 0.37, hydroxy value: 1053, specific gravity: 1.296) *9: Diglycerin #801, manufactured by Sakamoto Yakuhin Kogyo Co., Ltd. *10: Polyglycerin #310, manufactured by Sakamoto Yakuhin Kogyo Co., Ltd. (q/p = 0.79, r/p = 0.64, hydroxyl value: 1070, specific gravity: 1.270) *11: Polyglycerin #750, manufactured by Sakamoto Yakuhin Kogyo Co., Ltd. (q/p = 0.61, r/p = 1.22, hydroxyl value: 890, specific gravity: 1.263) *12: EMAL 125A, manufactured by Kao Corporation (25% by mass aqueous solution) *13: AMPHITOL 20AB, manufactured by Kao Corporation (30% by mass aqueous solution) *14: PENETOL GE-ID, manufactured by Kao Corporation (90% by mass aqueous solution) *15: AMIZOL CME, manufactured by Kawaken Fine Chemicals Co., Ltd. *16: EMULGEN 116, manufactured by Kao Corporation. | | | | | | | | |

As shown in Table 3, according to the shampoo of the present invention, high hair styling properties could be obtained by using polyglycerin having a specific polymerization degree distribution in combination with aromatic alcohol in low pH.

Moreover, when the hair cleaning composition of the present invention is used for hair, natural hand feeling can be provided from the hair.

### Examples 4 to 6

Hair shampoos shown in Table 4 were prepared, and the hair styling properties of each shampoo to the damaged hair were evaluated according to the methods and standards described above. However, the evaluation results of each hair styling properties in Examples 4 to 6 are given, not as an average of eight samples, but as evaluation results obtained from only a single sample.

**[Table 4]**

| Raw material (% by mass) | Example | | |
|---|---|---|---|
| | 4 | 5 | 6 |
| Polyglycerin (1) (*7) | 0.5 | 2.5 | 5.0 |
| Benzyl alcohol | 0.3 | 0.3 | 0.3 |
| Ammonium laureth sulfate solution (*12) | 5.2 | 5.2 | 5.2 |
| Lauramide propyl betaine solution (*13) | 3.33 | 3.33 | 3.33 |
| Isostearyl glyceryl ether solution (*14) | 0.67 | 0.67 | 0.67 |
| Cocamide MEA (*15) | 0.5 | 0.5 | 0.5 |
| Laureth-16 (*16) | 0.7 | 0.7 | 0.7 |
| Liquid malic acid (50% by mass) | 1.1 | 1.1 | 1.1 |
| Potassium hydroxide (48% by mass) | 0.23 | 0.23 | 0.23 |
| Ion exchange water | Balance | Balance | Balance |
| Total | 100 | 100 | 100 |
| pH (25°C) | 3.9 | 3.9 | 3.9 |
| Hair styling properties (n = 1) | 15 | 30 | 32 |

## Claims

1. A hair cleaning composition comprising components (A), (B), and (D), wherein the content of a component (C) is suppressed to 0% to 0.05% by mass, and the pH value of the hair cleaning composition is from 2.5 to 5.0;
(A): polyglycerin, in which with regard to the ratio among a total content (p) of triglycerin and tetraglycerin, a content (q) of diglycerin, and a total content (r) of pentaglycerin and higher order oligomers in the entire polyglycerin, q/p is less than 0.5 and r/p is less than 0.5;
(B): aromatic alcohol;
(C): cationic surfactant; and
(D): anionic surfactant.

2. The hair cleaning composition according to claim 1, wherein the content of the component (A) is from 0.1% to 10% by mass.

3. The hair cleaning composition according to claim 1 or 2, wherein the content of the component (B) is from 0.05% to 7.0% by mass.

4. The hair cleaning composition according to any one of claims 1 to 3, wherein the content of the component (C) is less than 0.03% by mass.

5. A method for treating hair, which comprises applying the hair cleaning composition according to any one of claims 1 to 4 to hair, washing the hair, rinsing it from the hair, and then drying the hair by natural drying or by blow-drying with hot air from a dryer.

6. A method for treating hair, which comprises applying the hair cleaning composition according to any one of claims 1 to 4 to hair, washing the hair, rinsing it from the hair, then drying the hair by natural drying or by blow-drying with hot air from a dryer, and then styling the hair by heating it using a heat generation type hair styling device.

7. Use of the hair cleaning composition according to any one of claims 1 to 4 for treating hair, which comprises applying the hair cleaning composition according to any one of claims 1 to 4 to hair, washing the hair, rinsing it from the hair, and then drying the hair by natural drying or by blow-drying with hot air from a dryer.

8. Use of the hair cleaning composition according to any one of claims 1 to 4 for re-styling hair, which comprises applying the hair cleaning composition according to any one of claims 1 to 4 to hair, washing the hair, rinsing it from the hair, then drying the hair by natural drying or by blow-drying with hot air from a dryer, and then styling the hair by heating it using a heat generation type hair styling device.
